# EUROPÄISCHE PATENTSCHRIFT

(11) **EP 0 514 789 B1**
(45) Veröffentlichungstag und Bekanntmachung des Hinweises auf die Patenterteilung: **11.09.1996**
(21) Anmeldenummer: 92108250.9
(22) Anmeldetag: 15.05.1992
(51) Int. Cl.: A61K 31/52

(54) **Die Verwendung von 1-(5-Oxohexyl)-3-methyl-7-n-propyl-xanthin in der Gefässchirurgie**
Use of 1-(5-oxohexyl)-3-methyl-7-n-propyl-xanthine in vascular surgery
Utilisation de 1-(5-oxohexyle)-3-méthyle-7-n-propyle-xanthine dans la chirurgie vasculaire

(30) Priorität: 23.05.1991 DE 4116799
(43) Veröffentlichungstag der Anmeldung: 25.11.1992
(73) Patentinhaber: HOECHST AKTIENGESELLSCHAFT, 65926 Frankfurt am Main (DE)
(72) Erfinder: Danielisova, Viera, Dr., CS-040 01 Kosice (CS); Kolvenbach, Ralf, Dr., W-4000 Düsseldorf (DE); Schubert, Hans-Peter, Prof. Dr., W-8031 Wörthsee-Steinebach (DE); Grome, John, Dr., W-6200 Wiesbaden (DE); Schneider, Ernst Jürgen, Dr., W-6277 Bad Camberg (DE)

(56) Entgegenhaltungen:
- US-A- 4 719 212
- STROKE, vol. 19, no. 12, December 1988, pages 1535-1539; J. DELEO et al.: "Protection against ischemic brain damage using propentofylline in gerbils"
- BIOCHEMICAL PHARMACOLOGY, vol. 39, no. 11, June 1990, pages 1813-1816, Pergamon Press plc, GB; I. SHINODA et al.: "Stimulation of nerve growth factor synthesis/secretion by propentofylline in cultured mouse astroglial cells"
- JOURNAL OF CEREBRAL BLOOD FLOW AND METABOLISM, vol. 7, no. 6, December 1987, pages 745-751, Raven Press, Ltd, New York, US; J. DELEO et al.: "Ischemia- induced neuronal cell death, calcium accumulation, and glial response in the hippocampus of the Mongolian gerbil and protection by propentofylline (HWA 285)"

## Beschreibung

Aus der Deutschen Patentschrift 2 330 742 ist bekannt, daß 1-(5-Oxohexyl)-3-methyl-7-n-propyl-xanthin (Verbindung 1) aufgrund ihrer gefäßerweiternden Wirkung bei geringer Toxizität zur Behandlung von Patienten geeignet ist, die an arteriellen Durchblutungsstörungen leiden. Dort werden ebenfalls Verfahren zur Herstellung dieser Verbindung beschrieben.

In der US 4,719,212 Patentschrift wird die Verwendung von 1-(5-Oxohexyl)-3-methyl-7-n-propyl-xanthin zur Behandlung von Gedächtnisstörungen beschrieben.

Ferner ist bekannt, daß 1-(5-Oxohexyl)-3-methyl-7-n-propyl-xanthin die Aufnahme von Adenosin in den Blutgefäßen des Rattengehirns inhibiert, die Mikrozirkulation in Blutgefäßen verbessert und einen Schutz vor der Entstehung von Gehirnödemen bietet (Cerebral Vascular Disease 5; Ed. J.S. Meyer et al., Excerpta Medica, 1985, Seiten 327 - 341).

Bei der Gefäßchirurgie handelt es sich um operative Behandlungen von Gefäßkrankheiten oder -verletzungen, wie sie vorallem bei der chirurgischen Therapie von koronarer, peripherer oder zerebraler Durchblutungsstörungen durchgeführt werden. Die wichtigsten Ursachen von Durchblutungsstörungen sind funktionelle, vasomotorische Gefäßstörungen, beispielsweise vasokonstriktorische Störungen z.B. primäres und sekundäres vasospastisches Syndrom oder vasodilatatorische Störungen z.B. Erythromelalgie, ferner obliterierende Gefäßkrankheiten im arteriellen Blutkreislauf, beispielsweise Arteriosklerose, diabetische Angiopathie, entzündliche Gefäßkrankheiten, wie Arteriitis oder Endangiitis, Thrombosen oder Embolien. Die arteriellen Gefäßkrankheiten können im Extremitätenbereich als periphere arterielle Verschlußkrankheit vorkommen oder viszeral, z.B. als Karotisverschluß, koronare Herzkrankheit, Nierenarterienstenose oder als Verschlüsse größerer Aortenäste vorkommen. Im venösen Blutkreislauf werden obliterierende Gefäßkrankheiten auch als tiefe Venenthrombose, Thrombophlebitis oder Phlebitis bezeichnet. Weitere Durchblutungsstörungen sind gefäßbedingte Mikrozirkulationsstörungen, Gefäßmißbildungen, Kompressionen oder traumatische Gefäßschäden.

Nachteile der gefäßchirurgischen Therapie sind operativ bedingte, teilweise oder vollständige, kurzzeitige Unterbrechungen der Blutzirkulation am Operationsort, die zu mehr oder weniger stark ausgeprägten Ausfallerscheinungen führen.

Bisher sind keine Arzneimittel bekannt, die es erlauben, die Zeit für eine symptomfreie Unterbrechung der Blutzirkulation zu verlängern oder die zu einer signifikanten Verminderung der durch die Unterbrechung der Blutzirkulation bedingten Nervenschädigungen führen. Eine erste Folge aufgrund der Unterbrechung der Blutzirkulation ist das zu Stoffwechselstörungen führende Sauerstoffmangelangebot in den davon betroffenen Arealen.

Es wurde nun gefunden, daß die Verbindung 1 geeignet ist, die Zeit für eine symptomfreie Unterbrechung der Blutzirkulation zu verlängern und die Nervenschädigungen, die nach Unterbrechung der Blutzirkulation auftreten, zu mindern.

Bei gefäßchirurgischen Operationen, wie beispielsweise Entfernung von Thromben, bei Transplantationen von Nieren oder Herz oder bei der Entfernung von Verschlüssen in Arterien oder Venen muß die Blutzirkulation teilweise oder ganz unterbrochen oder stark vermindert werden.

Je nach Dauer der Gefäßoperationen kann es dann zu mehr oder weniger starken Ausfallerscheinungen am zentralen Nervensystem kommen, die von leichten Hirnleistungsstörungen bis zu motorischen Ausfällen, wie z.B. Lähmungen, reichen können. Teilweise oder kurzzeitige Unterbrechungen der Blutzirkulation führen zu Beginn der Unterbrechung in der Regel nur zu reversiblen Schäden. Es verbleiben intakte Gewebe und Nerven, mit noch erhaltenem Energiemetabolismus, niedrigem extrazellulärem Kaliumgehalt und der Möglichkeit einer vollständigen Regeneration. Überraschenderweise zeigen Versuchstiere mit kurzzeitigen Gefäßverschluß der abdominalen Aorta nach Behandlung mit 1-(5-Oxohexyl)-3-methyl-7-n-propyl-xanthin eine schnelle Erholung des Energiemetabolismusses und Regeneration von neuronalen Defekten.

Die Erfindung betrifft daher die Verwendung von 1-(5-Oxohexyl)-3-methyl-7-n-propyl-xanthin und/oder von deren physiologisch verträglichen Salzen zur Herstellung von Arzneimitteln zur Regeneration von neuronalen Defekten, die nach Unterbrechung der Blutzirkulation auftreten und Erholung des Energiestoffwechsels der Nervenzellen.

Geeignete physiologisch verträgliche Salze von 1-(5-Oxohexyl)-3-methyl-7-n-propyl-xanthin sind beispielsweise Alkali-, Erdalkali- oder Ammoniumsalze, einschließlich solcher von physiologisch verträglichen organischen Ammoniumbasen.

Die Unterbrechung der Blutzirkulation kann durch Abschnürbinden oder Abschnürklemmen erfolgen um beispielsweise ein blutfreies Operationsfeld zu erhalten, sowie durch arterielle oder venöse Okklusion oder Verschluß aufgrund embolischer oder thrombotischer Verschlüsse in Blutgefäßen. Zur Unterbrechung der Blutzirkulation kommt es beispielsweise bei gefäßchirurgischen Operationen wie Entfernung von Thromben, bei Transplantationen von Organen wie Herz oder Niere sowie bei Verschlüssen in Arterien oder Venen, wie sie beispielsweise nach Schlaganfall, Gehirnschlag oder Herzinfarkt auftreten. Je nach Dauer der Blutzirkulationsunterbrechung kommt es zu mehr oder weniger starken Nervenschädigungen, die von leichten Nervenleistungsstörungen bis zum völligen Ausfall der Nerven, wie z.B. Lähmungen, reichen können. Als Symptome dieser Nervenschädigungen seien beispielsweise genannt: Membranpotentialstörungen der Nerven, geistige Verwirrung der Patienten, Orientierungslosigkeit, fehlende Ansprechbarkeit des Patienten, Gedächtnisstörungen, mangelnde Konzentrationsfähigkeit, motorische Störungen oder Lähmungen. Die Blutzirkulation kann völlig oder teilweise unterbrochen sein und dadurch bedingt werden mehr oder weniger starke Nervenschädigungen zu beobachten sein.

Die Herstellung von 1-(5-Oxohexyl)-3-methyl-7-n-propyl-xanthin erfolgt auf bekannte weise (DE 23 30 742). Die Verbindung 1-(5-Oxohexyl)-3-methyl-7-n-propyl-xanthin läßt sich beispielsweise herstellen, indem man ein Alkalimetallsalz der Verbindung der Formel I, 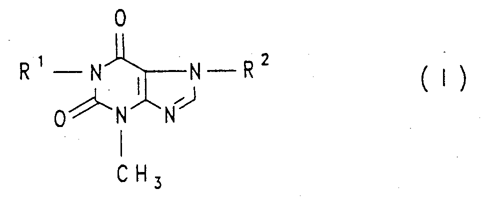 in der R¹ für Wasserstoff und R² für n-Propylgruppe steht in wäßrig-organischer Lösung mit einem Oxoalkylhalogenid der Formel II, 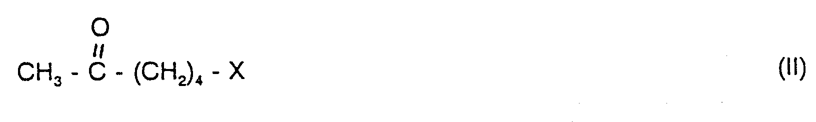 in der X für ein Halogenatom, wie Fluor, Chlor, Brom oder Jod steht, umsetzt.

Die erfindungsgemäßen Arzneimittel können oral, topisch, rektal, intravenös oder gegebenenfalls auch parenteral appliziert werden. Die Applikation kann vor, nach und während der Unterbrechung der Blutzirkulation erfolgen.

Aufgrund der pharmakologischen Eigenschaften der Verbindung 1 kann dieser Verbindung bei allen Operationen in der Klinik oder ambulanten Versorgung angewendet werden, in denen die Blutzirkulation an Geweben, Organen oder Extremitäten ganz oder teilweise unterbrochen wird. Ferner kann diese Verbindung auch bei Meniskusoperationen oder diagnostischen Eingriffen verwendet werden, wobei es zu einer völligen oder teilweisen Unterbrechung der Blutzirkulation im betroffenen Gewebe kommt. Insbesondere ist die Verbindung 1 zur Regeneration von neurohalen Defekten, die nach arteriell oder venös bedingten Verschlüssen der Blutgefäße auftreten, geeignet.

Geeignete feste oder flüssige galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.

Die Unterbrechung der Blutzirkulation sollte nicht viel länger als 240 Minuten dauern, bevorzugt 5 bis 120 Minuten, insbesondere 10 bis 30 Minuten. Die Zeit für die Unterbrechung der Blutzirkulation hängt im wesentlichen von der teilweisen oder vollständigen Unterbrechung ab und von den Geweben und Organen, die von der Blutzirkulation abgeschnitten werden. Die zeitlichen Grenzen der Unterbrechungszeit sind für den Fachmann einfach feststellbar.

Vorzugsweise werden die pharmazeutischen Präparate in Dosierungseinheiten hergestellt und verabreicht, wobei jede Einheit als aktiven Bestandteil eine bestimmte Dosis der Verbindung 1 und/oder mindestens ein physiologisch verträgliches entsprechendes Salz der Verbindung 1 enthält. Bei festen Dosierungseinheiten, wie Tabletten, Kapseln, Dragees oder Suppositorien, kann diese Dosis bis zu etwa 300 mg, bevorzugt jedoch etwa 10 bis 100 mg betragen.

Für die Behandlung eines Patienten (70 kg), der sich einer gefäßchirurgischen Behandlung unterziehen muß, ist vor, während und nach der Operation eine Dosis von 400 bis 1200 mg pro Tag und Patient der Verbindung 1 und/oder der entsprechenden Salze der Verbindung 1 am Menschen indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich kann die Verbindung 1 und/oder die entsprechenden Salze der Verbindung 1 bei der Herstellung der vorgenannten galenischen Zubereitungsformen auch zusammen mit anderen geeigneten Wirkstoffen, beispielsweise Wirkstoffen, die freie Sauerstoffradikale abfangen, z.B. 1,5-Dihydro-4H-pyrazolo(3,4-d)pyrimidin-4-on oder dem Enzym Superoxiddismutase, formuliert werden.

### Pharmakologische Prüfungen und Ergebnisse

Die Wirksamkeit der erfindungsgemäßen Arzneimittel wurde an Kaninchen untersucht, deren abdominale Aorta kurzzeitig abgeklemmt wurde. Dadurch wird eine Situation modellliert, wie sie der bei einer Gefäßoperation entspricht, also ein chirurgisches Modell, das im Gegensatz zur bilateralen Carotisokklusion am Gerbil oder der 4-Gefäßokklusion an der Ratte zu leichteren neuronalen Defekten führt. Die Wirkung der erfindungsgemäßen Arzneimittel auf die Erholung des Energiestoffwechsels und die Regeneration von neuronaler Defekte wurde beobachtet.

### Beispiel 1

### A) Operativer Eingriff

54 erwachsene Kaninchen (Gewicht 2,5 - 3 kg) wurden mit einer intravenösen Injektion von Pentobarbital (5-Ethyl-5-phenyl-2,4,6-trioxohexahydropyrimidin, Sigma) (30 mg/kg) betäubt. Ischämie wurde durch das Abklemmen der abdominalen Aorta auf der Höhe der linken zur Niere führenden Arterie für 20 oder 30 Minuten induziert.

Nach 20 Minuten Ischämie wurden je 6 Versuchstiere jeweils 1 mg/kg Verbindung 1, 5 mg/kg Verbindung 1, 10 mg/kg Verbindung 1 und 20 mg/kg Verbindung 1 intravenös injiziert. Nach 30 Minuten Ischämie wurden je 6 Versuchstieren jeweils 10 mg/kg und 20 mg/kg Verbindung 1 intravenös injiziert. Die Verbindung 1 wurde sofort nach dem Lösen der Aortaabklemmung verabreicht.

### B) Arterielle Blutanalyse

Während des obengenannten Eingriffs (A; 20 Min. Blutstromunterbrechung) wurde der pH und der Sauerstoff- und Kohlendioxidpartialdruck in der linken Schenkelarterie gemessen. Die Tabelle 1 zeigt die Ergebnisse. Bei den Kontrolltieren wurde die Aorta ebenfalls abgeklemmt, aber kein erfindungsgemäßes Arzneimittel gegeben.

**Tabelle 1**

| pH-Wert,Sauerstoffpartialdruck (pO₂) und Kohlendioxidpartialdruck (pCO₂) im arteriellen Blut von Kaninchen | | | | |
|---|---|---|---|---|
| | Kontrolltiere ohne Verbindung 1 | Verbindung 1 (mg/kg i.v.) | | |
| | | 5 | 10 | 20 |
| vor Blutstromunterbrechung | | | | |
| pH | 7.38±0.03 | 7.47±0.07 | 7.36±0.06 | 7.41±0.03 |
| pCO₂ (kPa) | 4.40±0.10 | 4.94±0.06 | 4.75±0.12 | 5.26±0.05 |
| pO₂ (kPa) | 13.70±0.20 | 10.17±0.47 | 9.41±0.15 | 10.98±0.06 |

| während der Blutstromunterbrechung | | | | |
|---|---|---|---|---|
| pH | 7.44±0.02 | 7.48±0.02 | 7.41±0.12 | 7.44±0.02 |
| pCO₂ (kPa) | 5.10±0.20 | 4.99±0.16 | 4.55±0.40 | 5.11±0.11 |
| pO₂ (kPa) | 12.80±0.50 | 10.08±0.06 | 9.51±0.05 | 9.80±0.12 |

| 5 Min Rezirkulation | | | | |
|---|---|---|---|---|
| pH | 7.43±0.01 | 7.51±0.03 | 7.46±0.01 | 7.43±0.02 |
| pCO₂ (kPa) | 5.20±0.20 | 4.84±0.02 | 4.58±0.02 | 5.23±0.03 |
| pO₂ (kPa) | 13.20±0.40 | 10.04±0.04 | 9.48±0.12 | 12.25±0.08 |

| 60 Min Rezirkulation | | | | |
|---|---|---|---|---|
| pH | 7.42±0.03 | 7.47±0.04 | 7.50±0.01 | 7.45±0.01 |
| pCO₂ (kPa) | 5.30±0.10 | 5.14±0.03 | 4.80±0.03 | 5.25±0.05 |
| pO₂ (kPa) | 13.10±0.40 | 10.58±0.05 | 9.90±0.10 | 12.38±0.06 |
| i.v. = intravenös Durchschnittswerte (n=6) ± Varianz | | | | |

### C) Biochemische Analyse

Rückenmark wurde nach Standardmethoden mit flüssigem Stickstoff eingefroren (Anderson et al., 1980, J. Neurosurg., 52, Seiten 387-391). Stücke des Rückenmarks (200 mg) wurden unterhalb des 5. Wirbels präpariert und nur das Rückenmarksgewebe wurde in flüssigem Stickstoff pulverisiert und Adenin-Nukleotide, Glucose und Lactat wurden mit Methanol-HCl und HClO₄ extrahiert (Folbergrova et al., 1974, Brain Res., 80, Seiten 265-279) und fluorometrisch bestimmt (Lowry und Passonneau, 1972, Academic Press, New York, Seite 291 ff.). Die Konzentrationsangaben beziehen sich auf mmol/kg Feuchtgewicht des Gewebes. Die Tabellen 2 und 3 fassen die Ergebnisse zusammen.

**Tabelle 2**

| Wirkungen der Verbindung 1 auf die Erholung von Rückenmarksgewebe des Kaninchens nach 20 Minuten Ischämie, gemessen nach 4 Tagen | | | | | | |
|---|---|---|---|---|---|---|
| | Kontrolltiere ohne Ischämie | Verbindung 1 (mg/kg i.v.) | | | | |
| | | 0 | 1 | 5 | 10 | 20 |
| ATP | 2.334±0.104 | 0.523±0.038 | 1.434±0.239 | 1.798±0.152 | 2.344±0.097 | 2.362±0.106 |
| Ad | 2.635±0.084 | 0.828±0.031 | 1.836±0.205 | 2.166±0.139 | 2.729±0.069 | 2.754±0.112 |
| Glucose | 2.573±0.155 | 0.966±0.027 | 1.641±0.193 | 1.640±0.147 | 2.278±0.085 | 2.535±0.110 |
| Lactat | 1.553±0.049 | 2.521±0.094 | 2.740±0.237 | 2.389±0.328 | 1.610±0.170 | 1.690±0.014 |
| Durchschnittswerte in mmol/kg Feuchtgewicht (n = 6) ± Varianz ATP Adenosintriphosphat Ad Adenin-Nukleotide | | | | | | |

**Tabelle 3**

| Wirkungen der Verbindung 1 auf die Erholung von Rückenmarksgewebe des Kaninchens nach 30 Minuten Ischämie, gemessen nach 4 Tagen | | | | |
|---|---|---|---|---|
| | Kontrolltiere ohne Ischämie | Verbindung 1 (mg/kg i.v.) | | |
| | | 0 | 10 | 20 |
| ATP | 2.334±0.104 | 0.539±0.105 | 0.677±0.106 | 0.993±0.106 |
| Ad | 2.635±0.084 | 0.990±0.106 | 1.134±0.073 | 1.669±0.120 |
| Glucose | 2.573±0.155 | 0.685±0.086 | 0.629±0.087 | 1.240±0.210 |
| Lactat | 1.553±0.049 | 3.232±0.155 | 2.436±0.219 | 1.781±0.139 |
| Einheiten der Werte wie in Tabelle 2 | | | | |

### D) Neurologische Beobachtungen

Die neurologischen Funktionen wurden nach der postoperativen Erholung beurteilt. Die Beurteilung der neurologischen Defekte erfolgt in 3 Gruppen:

### Gruppe 0:

Keine neurologischen Defekte; die Tiere hoppeln normal und schrecken vor visuellen Drohungen zurück.

### Gruppe 1:

Die Tiere ziehen die Hinterläufe hinter sich her und reagieren annormal auf visuelle Drohungen; leichte bis schwere Fehlfunktionen von Darm und Blase wurden mit einbezogen.

### Gruppe 2:

Völlige Lähmung der Hinterläufe und keine Reaktion auf Schmerzzufügung; starke Schädigung der Darm- und Blasenfunktionen.

Die Ergebnisse der neurologischen Beobachtungen der Versuchstiere mit 20 Min. Ischämie und 4 Tagen Erholung werden in Tabelle 4 zusammengefaßt und die Ergebnisse mit 30 Min Ischämie und 4 Tagen Erholung werden in Tabelle 5 gezeigt.

**Tabelle 4**

| Versuchstiere mit 20 Minuten Ischämie und 4 Tagen Erholung. Die Zahlen geben die Anzahl der Tiere wieder, die in die entsprechende Gruppe eingeordnet wurden | | | |
|---|---|---|---|
| | Gruppe 0 | Gruppe 1 | Gruppe 2 |
| Kontrolltiere ohne Verbindung 1 | - | 1 | 5 |
| 1 mg/kg Verbindung 1 | 1 | 3 | 2 |
| 5 mg/kg Verbindung 1 | 1 | 4 | 1 |
| 10 mg/kg Verbindung 1 | 5 | 1 | - |
| 20 mg/kg Verbindung 1 | 5 | 1 | - |

**Tabelle 5**

| Versuchstiere mit 30 Minuten Ischämie und 4 Tagen Erholung | | | |
|---|---|---|---|
| | Gruppe 0 | Gruppe 1 | Gruppe 2 |
| Kontrolltiere ohne Verbindung 1 | - | - | 6 |
| 10 mg/kg Verbindung 1 | - | 1 | 5 |
| 20 mg/kg Verbindung 1 | - | 5 | 1 |

### Beispiel 2

### Herstellung von 1-(5-Oxohexyl)-3-methyl-7-n-propyl-xanthin (Verbindung 1)

437,2 g 3-Methyl-7-propylxanthin, in einem Gemisch aus 240 g Methanol und 321 g Wasser suspendiert, werden bei erhöhter Temperatur mit 160 g 50 %iger Natronlauge in Lösung gebracht, anschließend bei Siedetemperatur mit 358 g 1-Bromhexanon-(5) versetzt und 4 1/2 Stunden am Rückfluß erhitzt. Nach dem Abkühlen wird nicht umgesetztes 3-Methyl-7-propylxanthin abgetrennt und der Alkohol abdestilliert. Die wäßrige Lösung wird mit Natronlauge auf pH 11 gestellt und mit Methylenchlorid extrahiert. Aus dem Rückstand der Methylenchloridlösung wird nach dem Umkristallisieren aus 5,2 l Diisopropylether 1-(5-Oxohexyl)-3-methyl-7-propylxanthin vom Schmelzpunkt 69 - 70°C in etwa 90 %iger Ausbeute (bezogen auf umgesetztes 3-Methyl-7-propylxanthin) erhalten.

## Patentansprüche

1. Verwendung von 1-(5-Oxohexyl)-3-methyl-7-n-propyl-xanthin oder mindestens einem von deren physiologisch verträglichen Salze zur Herstellung von Arzneimitteln zur Regeneration von neuronalen Defekten, die nach Unterbrechung der Blutzirkulation auftreten und Erholung des Energiestoffwechsels der Nervenzellen.

2. Verwendung gemäß Anspruch 1, dadurch gekennzeichnet, daß die Unterbrechung der Blutzirkulation durch eine gefäßchirurgische Operation verursacht wird und die Zeit der Unterbrechung von 5 bis 120 Minuten beträgt.

3. Verwendung gemäß Anspruch 1 oder 2, dadurch gekennzeichnet, daß die Unterbrechung der Blutzirkulation durch arteriell oder venös bedingte Verschlüsse der Blutgefäße verursacht wird.

4. Verwendung gemäß einem oder mehreren der Ansprüche 1 bis 3, dadurch gekennzeichnet, daß die Unterbrechung der Blutzirkulation nach einer gefäßchirurgischen Operation auftritt.

5. Verwendung gemäß einem oder mehreren der Ansprüche 1 bis 4, dadurch gekennzeichnet, daß die Applikation vor, nach oder während einer gefäßchirurgischen Operation erfolgt, bei der es zu einer Unterbrechung der Blutzirkulation kommt.

6. Verwendung gemäß Anspruch 2, dadurch gekennzeichnet, daß die Unterbrechung von 10 bis 30 Minuten beträgt.

## Claims

1. The use of 1-(5-oxohexyl)-3-methyl-7-n-propylxanthine or at least one of its physiologically tolerated salts for the preparation of pharmaceuticals for the regeneration of neuronal defects which occur after interruption of the blood circulation and recovery of the energy metabolism of the nerve cells.

2. The use as claimed in claim 1, wherein the interruption of the blood circulation is caused by a vascular surgical operation and the time of the interruption is from 5 to 120 minutes.

3. The use as claimed in claim 1 or 2, wherein the interruption of the blood circulation is caused by arterial or venous occlusions of the blood vessels.

4. The use as claimed in one or more of claims 1 to 3, wherein the interruption of the blood circulation occurs after a vascular surgical operation.

5. The use as claimed in one or more of claims 1 to 4, wherein administration takes place before, after or during a vascular surgical operation in which there is an interruption of the blood circulation.

6. The use as claimed in claim 2, wherein the interruption is for from 10 to 30 minutes.

## Revendications

1. Utilisation de la 1-(5-oxohexyl)-3-méthyl-7-*n*-propyl-xanthine ou au moins d'un de ses sels physiologiquement acceptables pour la préparation de médicaments pour la régénération de dommages neuronaux apparaissant à la suite de l'interruption de la circulation du sang et rétablissement du métabolisme énergétique des cellules nerveuses.

2. Utilisation selon la revendication 1, caractérisée en ce que l'interruption de la circulation sanguine est causée par une opération chirurgicale vasculaire et que la durée de l'interruption est de 5 à 120 min.

3. Utilisation selon la revendication 1 ou 2, caractérisée en ce que l'interruption de la circulation sanguine est causée par une occlusion artérielle ou veineuse des vaisseaux sanguins.

4. Utilisation selon une ou plusieurs des revendications 1 à 3, caractérisée en ce que l'interruption de la circulation sanguine apparait après une opération chirurgicale vasculaire.

5. Utilisation selon une ou plusieurs des revendications 1 à 4, caractérisée en ce que l'administration est effectuée avant, après ou pendant l'opération chirurgicale vasculaire au cours de laquelle intervient une interruption de la circulation sanguine.

6. Utilisation selon la revendication 2, caractérisée en ce que l'interruption dure de 10 à 30 min.
